# EUROPÄISCHE PATENTSCHRIFT

(11) **EP 1 681 145 B1**
(45) Veröffentlichungstag und Bekanntmachung des Hinweises auf die Patenterteilung: **17.09.2008**
(21) Anmeldenummer: 05027812.6
(22) Anmeldetag: 19.12.2005
(51) Int. Cl.: B27K 5/00

(54) **Verfahren und Mittel zur Verbesserung der Tränkmittelaufnahme und -verteilung in Hölzern**
Procedure and composition for the improvement of the uptake and distribution of soaking compositions in woods
Procédé et composition pour l'amélioration de la prise et la distribution de compositions de trempage en bois

(30) Priorität: 18.01.2005 DE 102005002366
(43) Veröffentlichungstag der Anmeldung: 19.07.2006
(73) Patentinhaber: EMPA Eidgenössische Materialprüfungs- und Forschungsanstalt, 8600 Dübendorf (CH)
(72) Erfinder: Schwarze, Francis W.M.R., Dr. PD, 5400 Baden (CH)
(74) Vertreter: Tetzner, Michael

(56) Entgegenhaltungen:
- WO-A-03/080812
- US-A- 4 698 305
- US-A1- 2002 096 273
- US-B1- 6 475 566
- F.W.M.R. SCHWARZE, H. LANDMESSER: "Preferential Degradation of Pit Membranes within Tracheids by Basidiomycete Physisporinus Vitreus" HOLZFORSCHUNG, Bd. 54, September 2000 (2000-09), Seiten 461-462, XP009066275 ISSN: 0453-3402
- HEIKKI KOTIRANTA: "Physisporinus rivulosis, an interesting polypore species" KARSTENIA, SOCIETAS MYCOLOGICA FENNICA, HELSINKI, FI, Bd. 25, 1985, Seiten 66-69, XP002967351 ISSN: 0453-3402

## Beschreibung

Die Erfindung betrifft ein Verfahren zur Verbesserung der Tränkmittelaufnahme und -verteilung in Hölzern sowie ein Verfahren zur Behandlung von Holz.

Die Fichte ist als schnell nachwachsender Baum eine der wichtigsten Baumarten in Mitteleuropa. Die geringe Dauerhaftigkeit des Fichtenholzes hat aber zur Folge, dass die Einsatzbereiche des Holzes stark eingeschränkt sind. Zwar kann das Fichtenholz durch eine chemische Holzschutzmittelbehandlung beständiger gemacht werden, allerdings ist das Fichtenholz nur schwierig ausreichend tief mit Holzschutzmitteln tränkbar.

Verantwortlich für diese schlechte Imprägnierbarkeit sind vor allem die im getrockneten Holz verschlossenen "Hoftüpfel", die im lebenden Baum als Verbindungswege für Flüssigkeiten zwischen aneinandergrenzenden Zellen dienen. Die Tüpfelmembran (Tori) der Hoftüpfel setzt sich aus den beiden Primärwänden der miteinander in Verbindung stehenden Tüpfel und der dazwischen liegenden Mittellamelle zusammen. Die zentralen verdickten Bereiche der Tüpfelmembranen des Splintholzes bestehen hauptsächlich aus Pektin, während Cellulose und Hemicellulose nur in geringen Konzentrationen vorhanden sind. Bei der Verkernung des Holzes werden dann in den Tori auch polyphenolische Substanzen mit Lignincharakter eingelegt. Hierbei wird die Öffnung der Hoftüpfelkammer (Porus) irreversibel verschlossen. Dieser Tüpfelverschluss hat zur Folge, dass die Tränkbarkeit von Fichtenkernholz selbst unter Anwendung großtechnischer Imprägnierverfahren erschwert ist.

Die Tatsache, dass Fichtenkernholz nicht tränkbar ist, hat zur Folge, dass eine Holzimprägnierung im Wesentlichen nur im Bereich des Splintholzes stattfindet, wobei sehr hohe Einbringmengen nur in den äußeren Splintholzzonen, aber nicht in den anderen Splintholzzonen verzeichnet werden. Diese ungleichmäßige Verteilung der Holzschutzmittel über den Querschnitt imprägnierter Bauteile ist sowohl aus ökonomischen als auch aus ökologischen Gründen bedenklich.

Man hat auch schon Versuche unternommen, die Imprägnierbarkeit von Fichtenholz durch eine enzymatische Vorbehandlung zu verbessern. Das zugrundeliegende biotechnische Verfahren basierte auf der abbauenden Wirkung ausgewählter spezifischer Enzyme bzw. Enzymgemische auf bestimmte Komponenten der Hoftüpfel, welchen den Flüssigkeitstransport im getrockneten Fichtenholz limitieren. Hierbei konnte sowohl die Eindringtiefe als auch die Aufnahmemenge des Holzschutzmittels gesteigert werden. Als am wirkungsvollsten zeigten sich Mischpräparate mit verschiedenen Pectinase-, Cellulase- und Hemicellulase Aktivitäten. Der Nachteil der Enzymbehandlungen besteht jedoch in der geringen Stabilität der Enzyme und dem Zeit- sowie Kostenaufwand der Behandlung. Dieses Verfahren hat daher keine wirtschaftliche Anwendung gefunden.

Weit verbreitet ist daher nach wie vor die mechanische Vorbehandlung durch Perforation der Mantelzonen mit Schlitzscheiben oder Bohrern. Ein zufrieden stellender Schutz von Schnitt- und Rundholz erfordert aber einen großen technischen Aufwand und ist nur durch anschließende Imprägnierung im Druck-Vacuum-Verfahren möglich. Durch dieses Verfahren wird aber im Wesentlichen nur das Splintholz besser imprägniert, nicht aber das Kernholz.

In der US 6,475,566 wird ein Verfahren zur Verbesserung der Impärgnierbarkeit von Holz offenbart, bei dem ein Pilz aus der Gruppe der Basidiomyceten, Ascomyceten und Deuteromyceten ausgewählt wird. Weiterhin ist durch F.W.M.R. Schwarze, H. Landmesser: "Preferential Degradation of Pit Membranes within Tracheids by Basidiomycete Physisporinus Vitreus", Holzforschung, Bd. 54, September 2000 (2000-09), Seiten 461-462 der Abbau der Tori bei feuchtem Holz durch den Pilz Physisporinus vitreus bekannt.

Der Erfindung liegt daher die Aufgabe zugrunde ein neues Verfahren zur Verbesserung der Tränkmittelaufnahme und -verteilung im Kernholzbereich von Hölzern sowie ein neues Verfahren zur Behandlung von Holz anzugeben.

Erfindungsgemäß wird diese Aufgabe durch die Merkmale der Ansprüche 1 und 8 gelöst.

Weitere Ausgestaltungen der Erfindung sind Gegenstand der Unteransprüche.

Das erfindungsgemäße Verfahren zur Verbesserung der Tränkmittelaufnahme und - verteilung in Hölzern, insbesondere Nadelhölzern, besteht darin, dass das zu behandelnde Holz mit dem Pilz Physisporinus vitreus derart in Kontakt gebracht wird, dass die Hyphen des Pilzes das Splint- und Kernholz gleichmäßig besiedeln und auch die lignifizierten Tüpfelmembranen im Kernholz abgebaut werden, wobei die Behandlungszeit so gewählt wird, dass die Festigkeit des Holzes nicht wesentlich verschlechtert wird.

Bei dem erfindungsgemäßen Verfahren zur Behandlung von Holz, insbesondere Nadelholz wird das Holz zunächst über einen bestimmten Zeitraum mit dem Pilz Physisporinus vitreus und dann anschließend mit einem Tränkmittel, insbesondere mit einem Holzschutzmittel oder einem Holzveredelungsmittel, behandelt.

Das erfindungsgemäße Mittel zur Verbesserung der Tränkmittelaufnahme und - verteilung in Hölzern, insbesondere Nadelhölzern enthält den Pilz Physisporinus vitreus.

Im Gegensatz zu Bakterien und Enzymen bietet der Einsatz des Pilzes Physisporinus vitreus im Zusammenhang mit der gezielten Verbesserung der Wegsamkeit wesentliche Vorteile. Die Hyphen des Pilzes Physisporinus vitreus können das Splint- und Kernholz gleichmäßig besiedeln und sind in der Lage, die lignifizierten Tüpfelmembranen im Kernholz abzubauen. Im Gegensatz zu anderen holzzersetzenden Pilzen hat der Pilz Physisporinus vitreus den Vorteil, dass bei geeigneter Wahl der Behandlungszeit die Festigkeit des Holzes nicht wesentlich verschlechtert wird.

Der als Weissfäuleerreger eingestufte Basidiomycet Physisporinus vitreus gehört zu den wenigen holzzersetzenden Pilzen, die die Fähigkeit besitzen, extrem feuchtes Holz abzubauen. Auffälligstes Merkmal des Holzzersetzungsmusters von Physisporinus vitreus an natürlich wie künstlich infiziertem Fichten- und Douglasienholz ist die bereits im Frühstadium der Holzzersetzung einsetzende starke Zerstörung der Hoftüpfel zwischen den Tracheiden.

Die der Erfindung zugrunde liegenden Studien haben gezeigt, dass der Pilz in der Lage ist, sehr feuchtes Holz stärker zu zersetzen als trockenes. Diese Tatsache steht offensichtlich in direktem Zusammenhang mit dem bevorzugten Hoftüpfelabbau im Initialstadium der Holzzersetzung. Es wird angenommen, dass Physisporinus vitreus durch den bevorzugten Hoftüpfelabbau im trockenen Holz indirekt eine Feuchtigkeitsaufnahme begünstigt und somit die Voraussetzung für eine Holzfeuchtigkeit induziert.

Bei dem durchgeführten Versuchen hat sich als vorteilhaft erwiesen, wenn die Anfangsfeuchte des zu behandelnden Holzes vor der Pilzbehandlung bei maximal 15%, vorzugsweise zwischen 10 und 15%, liegt. Zweckmäßigerweise wird während der Pilzbehandlung die Holzfeuchte des zu behandelnden Holzes überwacht. Prinzipiell sind die Anfangsfeuchte des zu behandelnden Holzes und die Einwirkdauer des Pilzes derart aufeinander abzustimmen, dass er während des Behandlungszeitraumes im wesentlichen nur die Tori und nicht die Zellwände des zu behandelnden Holzes zersetzt. Die Anfangsfeuchte des zu behandelnden Holzes und die Einwirkdauer des Pilzes sollten ferner derart aufeinander abgestimmt werden, dass sich die Schlagbiegefestigkeit des zu behandelnden Holzes um nicht mehr als 20% verschlechtert. Der Behandlungszeitraum sollte zwei bis sechs Wochen, vorzugsweise drei bis fünf Wochen dauern.

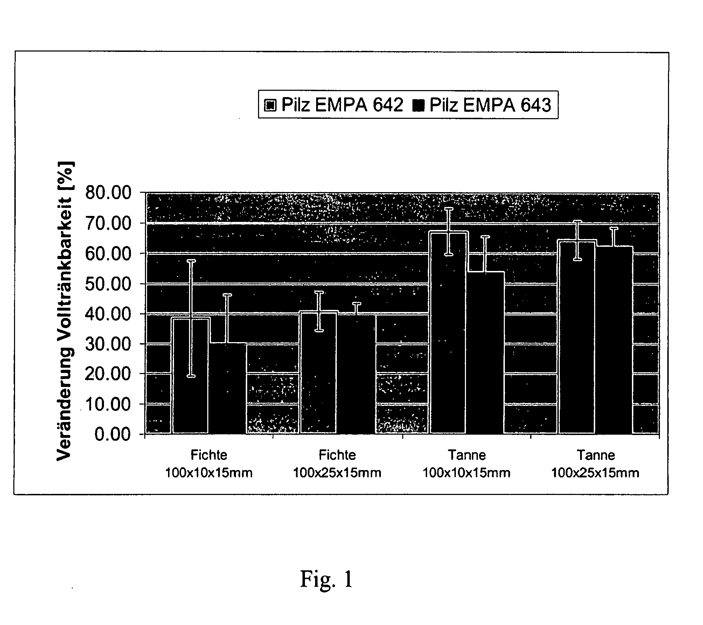

Die Wirkung des mit dem Pilz Physisporinus vitreus behandelten Holzes wird anhand der Fig. 1 und der nachfolgend beschriebenen Versuchsergebnisse gezeigt.

Für die Versuche wurden zwei Reinkulturen von Physisporinus vitreus (EMPA 642 und EPMA 643) in Kolleschalen aus 2,5% MEA angezüchtet. Unbehandelte, stirnseitig abgedichtete Kernholzproben wurden zur Erfassung der Volltränkbarkeit in endmineralisiertem Wasser bzw. mit Zugabe von blauem Farbpigment Neolan Glaucan E-A vor und nach der Pilzexposition vollgetränkt. Es wurden Holzproben mit möglichst großem Kernholzanteil ausgewählt. Zur Erfassung des Einflusses der

Mantelfläche/Querschnittsabmessungen wurden die Untersuchungen anhand von zwei verschiedenen Probenabmessungen durchgeführt:
a) 100 mm (Faserrichtung) x 10 mm x 15 mm (radial)
b) 100 mm (Faserrichtung) x 25 mm x 51 mm (radial)

Vor der Pilzexposition zur Bestimmung der ersten Volltränkbarkeit (Ausgangs-Volltränkbarkeit ohne Pilzeinwirkung) wurden Holzproben zweimal im Intervall von 24 h mit Nouvovern Emaillack (zwei Komponenten-Urethanlack der Fa. Mäder AG, Killwangen) abgedichtet. Die abgedichteten Holzproben wurden zur Bestimmung des Anfangsdarrgewichtes während 18-24 h im Trockenschrank bei 103°C darrgetrocknet. Anschließend wurden die Holproben bei einem Unterdruck von 7 mbar für 20 min. in entmineralisiertem Wasser evakuiert und nach zweistündiger Lagerung in Wasser, durch Wägen die aufgenommen Flüssigkeitsmenge bestimmt. Die Volltränkbarkeit in kg/m³ wurde anhand der Probendimensionen errechnet bzw. in Relation zur maximalen Volltränkbarkeit in Prozent (Absolut in %) angegeben.

Die Pilzexposition erfolgte in Kolleschalen über Glasbänkchen, sobald das Pilzmyzel den Nährboden komplett überwachsen hatte. Die Inkubation der Holzproben erfolgte bei 22 ± 1°C und 75 ± 5% rel. Luftfeuchtigkeit und für sechs und zwölf Wochen. Nach Entfernen der anhaftenden Pilzmyzelreste wurden die einzelnen Holzproben gewogen, um später den Feuchtigkeitsgehalt der Holzproben bei Pilzversuchsende, anhand des Enddarrgewichtes zu berechnen. Ein möglicher Gewichtsverlust durch Pilzangriff wurde anhand Anfangsdarr- und Enddarrgewicht erfasst, und mittels steriler Kontrollen (Nullwerte) korrigiert.

Nach der Pilzexposition zur Bestimmung der zweiten Volltränkbarkeit (verbesserte Volltränkbarkeit nach Pilzentwicklung) wurden die feuchten Holzproben vorerst während 48 h im Trockenschrank bei 4°C vorgetrocknet und anschließend ihre Hirnenden erneut einmal mit Nouvovern Emaillack abgedichtet. Anschließend erfolgte die zweite Volltränkung. Zu diesem Zweck wurden die Holzproben erneut während 18-24 h bei 103°C darrgetrocknet (Bestimmung des Enddarrgewichtes). Die erneut abgedichteten Hirnenden der pilzexponierten Holzproben wurden zusätzlich mechanisch abgedichtet, um einer Rissbildung entgegenzuwirken. Zu diesem Zweck wurden die Stirnseiten mit Weichsilikon überzogen und mit Metallschienen stirnseitig eingespannt und in entmineralisiertem Wasser bzw. unter Zugabe des Farbpigmentes vollgetränkt.

Nach sechs und zwölf Wochen Inkubationszeit konnte bereits eine deutliche Steigerung der Volltränkbarkeit an den pilzexponierten Kernholzproben beobachtet werden (Fig. 1). An Fichtenkernholzproben konnte eine Zunahme der Tränkbarkeit von 30-40%, an Tannenkernholz sogar von 50-60% ermittelt werden. Die größer dimensionierten Kernholzproben wiesen zum Teil eine höhere Steigerung der Volltränkbarkeit auf, als die kleineren Kernholzproben.

Bei der großtechnologischen Umsetzung der Oberflächenbehandlung von Schnittholz wird das getrocknete, insbesondere luftgetrocknete Schnittholz in ein Flüssigmedium getaucht, welches das Myzel von Physisporinus vitreus enthält. Nach zweiwöchiger Inkubationszeit bei einer Temperatur von 20-30°C und einer relativen Luftfeuchtigkeit von etwa 65% wird das Holz oberflächig vom Myzel gereinigt und mit dem Holzveredelungsmittel behandelt.

Alternativ zur oberflächlichen Reinigung kann das behandelte Holz auch sterilisiert werden, um den Pilz abzutöten. Hierfür wird das Schnittholz beispielsweise bei 0,6 bar und 25° mit Ethylenoxid in Kontakt gebracht. Das so behandelte Holz kann als eigenständiges Produkt vertrieben werden.

Durch die Pilzbehandlung mit Physisporinus vitreus kann die Tränkbarkeit des Holzes deutlich erhöht werden, wobei im Initialstadium der Holzzersetzung die Festigkeit des Holzes nicht maßgeblich beeinträchtigt wird. Der selektive Abbau der Tüpfelmembranen durch den Pilz ist somit hervorragend geeignet, um die Wegsamkeit des Holzes zu erhöhen und das Eindringen von Veredelungs- und Schutzmitteln, insbesondere von fungizid ausgestatteten Grundierungen und Lasuren, für die Oberflächenbehandlung zu verbessern. Die erhöhte Permeabilität des Holzes hat zur Folge, dass die Wirkstoffe tiefer in das Holz eindringen und dadurch die bläuefreie Zone erhöht werden kann. Eine größere bläuefreie Zone hat den Vorteil, dass die Entwicklung von Bläue- und Schimmelpilzen unterhalb der Anstrichsysteme stärker gehemmt und ein Aufreißen und Abplatzen des Deckanstriches verhindert wird.

Durch die verbesserte Tränkmittelaufnahme kann außerdem die Tränkmittelmenge über den zu schützenden Querschnitt homogener verteilt werden, wodurch die Kosten für die Imprägniermittel und zudem die Verfahrenskosten gesenkt werden. Darüber hinaus kann auch die Schutzbehandlung durch die erhöhte Tiefenwirkung verbessert werden. Umwelttechnische Vorteile ergeben sich außerdem dadurch, dass Auswaschungen aus den heute noch überimprägnierten Randzonen reduziert sind und der Stoff- und Energieeinsatz gesenkt wird.

Auch moderne Verfahren der Veredelung und Modifikation, z.B. Verkieselung/Hydrophobierung, Behandlung mit pflanzlichen Ölen, Actylisierung, Melaminharztränkung, usw., können ebenfalls durch die erhöhte Wegsamkeit des Holzes enorm optimiert werden.

Eine verbesserte Wegsamkeit des Kernholzes hat auch einen ökologischen Vorteil, da die Eindringmengen von Holzschutzmitteln über den gesamten Rundholzquerschnitt gleichmäßiger verteilt und somit in geringeren Konzentrationen bei reduziertem Aufwand eingebracht werden können. Bei erfolgreicher Umsetzung kann somit eine Vielzahl von Behandlungs- und Veredelungsprozessen von Holz, insbesondere Fichtenholz, effizienter und wertschöpfender gestaltet werden und dabei die wirtschaftliche Nutzung der wichtigsten Baumart im Mitteleuropa maßgeblich verbessert werden.

## Patentansprüche

1. Verfahren zur Verbesserung der Tränkmittelaufnahme und -verteilung in Hölzern, insb. Nadelhölzern, **dadurch gekennzeichnet, dass** das zu behandelnde Holz mit dem Pilz Physisporinus vitreus derart in Kontakt gebracht wird, dass die Hyphen des Pilzes das Splint- und Kernholz gleichmäßig besiedeln und auch die lignifizierten Tüpfelmembranen im Kernholz abgebaut werden, wobei die Behandlungszeit so gewählt wird, dass die Festigkeit des Holzes nicht wesentlich verschlechtert wird.

2. Verfahren nach Anspruch 1, **dadurch gekennzeichnet, dass** die Anfangsfeuchte des zu behandelnden Holzes vor der Pilzbehandlung bei maximal 15% liegt.

3. Verfahren nach Anspruch 1, **dadurch gekennzeichnet, dass** die Anfangsfeuchte des zu behandelnden Holzes und die Einwirkdauer des Pilzes derart aufeinander abgestimmt werden, dass der Pilz während des Behandlungszeitraumes im wesentlichen nur die Tori und nicht die Zellwände des zu behandelnden Holzes zersetzt.

4. Verfahren nach Anspruch 1, **dadurch gekennzeichnet, dass** die Pilzbehandlung 2 bis 6 Wochen, vorzugsweise 3 bis 5 Wochen dauert.

5. Verfahren nach Anspruch 1, **dadurch gekennzeichnet, dass** während der Pilzbehandlung die Holzfeuchte des zu behandelnden Holzes überwacht wird.

6. Verfahren nach Anspruch 1, **dadurch gekennzeichnet, dass** die Anfangsfeuchte des zu behandelnden Holzes und die Einwirkdauer des Pilzes derart aufeinander abgestimmt werden, dass sich die Schlagbiegefestigkeit des zu behandelnden Holzes um nicht mehr als 20% verschlechtert.

7. Verfahren nach Anspruch 1, **dadurch gekennzeichnet, dass** der Pilz nach einer vorgegebenen Behandlungszeit abgetötet.

8. Verfahren zur Behandlung von Holz, insbesondere Nadelholz, **dadurch gekennzeichnet, dass** zunächst die Tränkmittelaufnahme und -verteilung des Holzes gemäß einem oder mehreren der Ansprüche 1 bis 7 verbessert wird und anschließend das Holz mit einem Tränkmittel, insbesondere mit einem Holzschutzmittel oder einem Holzveredelungsmittel, behandelt wird.

## Claims

1. Method for improving the intake and distribution of impregnating compositions in woods, in particular softwoods, **characterised in that** the wood to be treated is brought into contact with the fungus Physisporinus vitreus in such a manner that the hyphae of the fungus colonise the sapwood and the heartwood to the same extent and also the lignin-containing pit membranes in the heartwood are broken-down, the treatment time being selected in such a manner that the strength of the wood is not significantly impaired.

2. Method according to claim 1, **characterised in that** the initial moisture level of the wood to be treated prior to the fungus treatment is a maximum of 15%.

3. Method according to claim 1, **characterised in that** the initial moisture level of the wood to be treated and the effective duration of the fungus are adapted to each other in such a manner that, during the treatment time, the fungus breaks down substantially only the tori and not the cell walls of the wood to be treated.

4. Method according to claim 1, **characterised in that** the fungus treatment lasts for a period of from 2 to 6 weeks, preferably from 3 to 5 weeks.

5. Method according to claim 1, **characterised in that,** during the fungus treatment, the wood moisture level of the wood to be treated is monitored.

6. Method according to claim 1, **characterised in that** the initial moisture level of the wood to be treated and the effective duration of the fungus are adapted to each other in such a manner that the impact strength of the wood to be treated is impaired by no more than 20%.

7. Method according to claim 1, **characterised in that** the fungus is killed after a predetermined treatment time.

8. Method for the treatment of wood, in particular softwood, **characterised in that** the impregnating composition intake and distribution of the wood is first improved in accordance with any one or more of claims 1 to 7 and, subsequently, the wood is treated with an impregnating composition, in particular with a wood protection agent or a wood finishing agent.

## Revendications

1. Procédé pour améliorer l'absorption et la diffusion de compositions d'imprégnation de bois, en particulier de conifères, **caractérisé en ce que** le bois à traiter est amené en contact avec le champignon Physisporinus vitreus de telle façon que le mycélium du champignon ensemence de la même façon l'aubier et le coeur du bois, et que les ponctuations lignifiées sont également attaquées dans le bois de coeur, la durée de traitement étant choisie de telle sorte que la résistance du bois ne soit pas sensiblement détériorée.

2. Procédé selon la revendication 1, **caractérisé en ce que** l'humidité de départ du bois à traiter, avant traitement par le champignon, est de 15 % maximum.

3. Procédé selon la revendication 1, **caractérisé en ce que** l'humidité de départ du bois à traiter et la période d'action du champignon sont ajustées l'une par rapport à l'autre de telle sorte que, pendant l'intervalle de traitement, le champignon ne décompose sensiblement que les tori, et pas les parois cellulaires du bois à traiter.

4. Procédé selon la revendication 1, **caractérisé en ce que** le traitement par le champignon dure de 2 à 6 semaines, de préférence de 3 à 5 semaines.

5. Procédé selon la revendication 1, **caractérisé en ce que**, pendant le traitement par le champignon, on surveille l'humidité du bois à traiter.

6. Procédé selon la revendication 1, **caractérisé en ce que** l'humidité de départ du bois à traiter et la période d'action du champignon sont ajustées l'une par rapport à l'autre de telle sorte que la résistance à la flexion au choc du bois à traiter n'est pas détériorée de plus de 20 %.

7. Procédé selon la revendication 1, **caractérisé en ce que** le champignon est tué après une période de traitement prédéterminée.

8. Procédé de traitement de bois, en particulier de conifères, **caractérisé en ce que** l'absorption et la diffusion de compositions d'imprégnation du bois est d'abord améliorée selon l'une ou plusieurs de revendications 1 à 7, et le bois est ensuite traité avec une composition d'imprégnation, en particulier une composition de protection du bois ou une composition d'amélioration du bois.
